# EUROPEAN PATENT APPLICATION

(11) **EP 4 445 850 A1**
(43) Date of publication of application: **16.10.2024**
(21) Application number: 22903620.7
(22) Date of filing: 09.12.2022
(51) Int. Cl.: A61B 17/32, A61B 18/12, A61B 18/14

(54) **DEVICE FOR OUTPUTTING DRIVE SIGNAL TO SURGICAL INSTRUMENT, AND SURGICAL SYSTEM**

(30) Priority: 09.12.2021 CN 202111497820
(71) Applicant: Shanghai Yichao Medical Devices Co., Ltd., Shanghai 201201 (CN); Qingdao Medbios Medical Technology Co., Ltd, Qingdao, Shandong 266114 (CN)
(72) Inventor: FENG, Qingyu, Qingdao, Shandong 266114 (CN); XU, Wangyang, Qingdao, Shandong 266114 (CN); GENG, Zhiqiang, Qingdao, Shandong 266114 (CN); SHI, Yiping, Qingdao, Shandong 266114 (CN); LIU, Weiqi, Qingdao, Shandong 266114 (CN)
(74) Representative: Petraz, Gilberto Luigi
(86) International application number: PCT/CN2022/138116
(87) International publication number: WO 2023/104203

(57) **Abstract**

An output device for outputting drive signals to a surgical instrument and a surgical system. The device includes an ultrasonic energy source and a high-frequency electric energy source located in the same casing, a signal port, an ultrasonic signal acquisition circuit, a high-frequency electric signal acquisition circuit, and a control module, and the control module is configured to obtain adjustment parameters based on the feedback signals, and control the ultrasonic energy source and the high-frequency electric energy source to output drive signals based on the adjustment parameters. The surgical instrument includes one or more of an ultrasonic-electric scalpel, an ultrasonic scalpel, a monopolar electrotome, and a bipolar electrotome. According to the technical solution of the embodiments of the present disclosure, the ultrasonic energy source and the high-frequency electric energy source are packaged in the same casing of the output device, and the output device can simultaneously drive both the ultrasonic surgical instrument and the high-frequency electrosurgical instrument, thereby saving the device space avoiding additional cost, facilitating control, and helping to improve surgical efficiency and achieve better surgical effects.

## Description

### Field of the Invention

The present disclosure relates to ultrasonic electrosurgical system for performing surgical operations and, more particularly, to a device for outputting drive signals to a surgical instrument, and a surgical system.

### Background of the Invention

Both an ultrasonic surgical instrument (or an ultrasonic scalpel for short) and a high-frequency electrosurgical instrument (or an electrotome for short) can be used for performing surgical operations. The ultrasonic scalpel has good cutting performance, but has poor blood coagulation performance during surgical procedures. According to the working mode, the electrotome is divided into the monopolar electrotome and the bipolar electrotome. The bipolar electrotome has good coagulation performance, but has poor cutting performance during surgical procedures. When the electrodes are set on the end of the ultrasonic scalpel, it can achieve the effects of both ultrasonic scalpel and bipolar electrotome, and such multifunctional ultrasonic surgical instrument is referred to as "ultrasonic-electric scalpel".

### Summary

### Technical Problem

The ultrasonic-electric scalpel, the ultrasonic scalpel, the monopolar electrotome, and the bipolar electrotome are needed for surgical operation, and several types of surgical instruments therein are often used during one surgery. At present, the energy output devices for driving these surgical instruments are independent devices, and so, these energy output devices not only occupy the limited space in the operating room, but also produce a lot of inconvenience in controlling these output devices when they are used, and even affect the operation effect. For example, an ultrasonic scalpel is driven by a separate ultrasonic output device, and a monopolar electrotome or a bipolar electrotome is driven by a separate high-frequency electric output device. When driving the ultrasonic-electric scalpel, it can be using the manner of cable-connecting an ultrasonic output device and a high-frequency electric output device, or using a integrated device with two energy sources, but in this case, an ultrasonic drive signal and a high-frequency electric drive signal output by the integrated device are superimposed, a special ultrasonic transducer is required to separate signal from the output so as to drive the ultrasonic-electric scalpel, and the ultrasonic transducer is a vulnerable part, this manner undoubtedly increases application costs.

### Solutions to the Problem

### Technical Solutions

In order to solve the problems in the related art, embodiments of the present disclosure provide an output device for outputting drive signals to a surgical instrument and a surgical system.

One aspect of the present disclosure provides an output device for outputting drive signals to a surgical instrument, comprising:
an ultrasonic energy source for generating an ultrasonic drive signal;
a high-frequency electric energy source for generating a high-frequency electric drive signal, wherein the ultrasonic energy source and the high-frequency electric energy source are located in the same casing;
a signal port for connecting to a surgical instrument, so as to provide the ultrasonic drive signal and/or the high-frequency electric drive signal to the surgical instrument;
an ultrasonic signal acquisition circuit, used for acquiring and processing signals in a connection circuit between the surgical instrument and the ultrasonic energy source, so as to obtain ultrasonic feedback signals, and providing the ultrasonic feedback signals to a control module, wherein the ultrasonic feedback signals comprise an ultrasonic voltage feedback signal and an ultrasonic current feedback signal;
a high-frequency electric signal acquisition circuit, used for acquiring and processing signals in a connection circuit between the high-frequency electric energy source and the surgical instrument, so as to obtain high-frequency electric feedback signals, and providing the high-frequency electric feedback signals to the control module, wherein the high-frequency electric feedback signals comprise a high-frequency voltage feedback signal and a high-frequency current feedback signal;
a control module configured to obtain adjustment parameters based on the ultrasonic feedback signals and the high-frequency electric feedback signals; and to control the ultrasonic energy source to output an ultrasonic drive signal and the high-frequency electric energy source to output a high-frequency electric drive signal based on the adjustment parameters; wherein the adjustment parameters comprise a power parameter and a frequency parameter, the power parameter is used for adjusting the power of the ultrasonic drive signal output by the ultrasonic energy source and the power of a high-frequency electric drive signal output by the high-frequency electric energy source, the frequency parameter is used for adjusting the frequency of the ultrasonic drive signal output by the ultrasonic energy source and/or the frequency of the high-frequency electric drive signal output by the high-frequency electric energy source,
wherein the surgical instrument comprises one or more of an ultrasonic-electric scalpel, an ultrasonic scalpel, a monopolar electrotome, and a bipolar electrotome.

According to an embodiment of the present disclosure, the control module obtains adjustment parameters based on the ultrasonic feedback signals and the high-frequency electric feedback signals, including:
obtains an acoustic impedance based on the ultrasonic feedback signals, and obtains an electric impedance based on the high-frequency electric feedback signal;
matches the acoustic impedance and/or the electric impedance with impedance data to determine a tissue type;
based on the tissue type, matches the acoustic impedance and/or the electric impedance with the impedance data to obtain a power parameter.

According to an embodiment of the present disclosure, the control module is further configured to:
obtain a first phase difference based on the ultrasonic voltage feedback signal and the ultrasonic current feedback signal;
obtain a second phase difference based on the high-frequency voltage feedback signal and the high-frequency current feedback signal;
based on the tissue type, match the acoustic impedance and/or the electric impedance with the impedance data, and obtain the power parameter according to the first phase difference and the second phase difference..

According to an embodiment of the present disclosure, the control module is further configured to control the duration of output signals of the ultrasonic energy source and the high-frequency electric energy source.

According to an embodiment of the present disclosure, the control module further comprises:
a first processing module, configured to perform digital filtering and digital operation on the ultrasonic feedback signals to obtain an acoustic impedance;
a second processing module, configured to perform digital filtering and digital operation on the high-frequency electric feedback signals to obtain an electric impedance.

According to an embodiment of the present disclosure, wherein,
the ultrasonic energy source comprises: an ultrasonic frequency regulation module, an ultrasonic power regulation module and an ultrasonic signal generator;
the high-frequency electric energy source comprises: a high-frequency electric frequency regulation module, a high-frequency electric power regulation module and a high-frequency electric signal generator.

According to an embodiment of the present disclosure, the ultrasonic signal acquisition circuit comprises: a first filter module, a first differential amplification module, a second filter module, a first automatic-gain-control module, and a first analog-to-digital conversion module.

According to an embodiment of the present disclosure, the high-frequency electric signal acquisition circuit comprises: a third filter module, a second differential amplification module, a fourth filter module, a second automatic-gain-control module, and a second analog-to-digital conversion module.

According to an embodiment of the present disclosure, the signal port is further configured to receive a manual switch signal of the surgical instrument and providing the manual switch signal to the control module;
and the control module is further configured to control the power of an output signal of the ultrasonic energy source and/or the power of an output signal of the high-frequency electric energy source according to the manual switch signal and the adjustment parameters.

According to an embodiment of the present disclosure, the device further comprises an input module for receiving parameter settings;
and the control module is further configured to control the power of an output signal of the ultrasonic energy source and/or power of an output signal of the high-frequency electric energy source, according to the parameter settings and the adjustment parameters.

Another aspect of the present disclosure provides a surgical system comprising an output device and a surgical instrument,
wherein the output device comprises:
an ultrasonic energy source for outputting an ultrasonic drive signal;
a high-frequency electric energy source for outputting a high-frequency electric drive signal, wherein the ultrasonic energy source and the high-frequency electric energy source are located in the same casing;
a signal port, used for connecting to a surgical instrument, so that the surgical instrument obtains the ultrasonic drive signal and/or the high-frequency electric drive signal;
an ultrasonic signal acquisition circuit, used for acquiring and processing signals in a connection circuit between the ultrasonic energy source and the surgical instrument, so as to obtain ultrasonic feedback signals, and provide the ultrasonic feedback signals to a control module, wherein the ultrasonic feedback signals comprise an ultrasonic voltage feedback signal and an ultrasonic current feedback signal;
a high-frequency electric signal acquisition circuit, used for acquiring and processing signals in a connection circuit between the high-frequency electric energy source and the surgical instrument, so as to obtain high-frequency electric feedback signals, and providing the high-frequency electric feedback signals to a control module, wherein the high-frequency electric feedback signals comprise a high-frequency voltage feedback signal and a high-frequency current feedback signal;
a control module configured to obtain adjustment parameters based on the ultrasonic feedback signals and the high-frequency electric feedback signals; and to control the ultrasonic energy source to output an ultrasonic drive signal and to control the high-frequency electric energy source to output a high-frequency electric drive signal based on the adjustment parameters; wherein the adjustment parameters comprise a power parameter and a frequency parameter, the power parameter being used for adjusting the power of the ultrasonic drive signal output by the ultrasonic energy source and the power of the high-frequency electric drive signal output by the high-frequency electric energy source, the frequency parameter being used for adjusting the frequency of the ultrasonic drive signal output by the ultrasonic energy source and/or the frequency of the high-frequency electric drive signal output by the high-frequency electric energy source;
the surgical instrument comprises an ultrasonic-electric scalpel and a monopolar electrotome, wherein the ultrasonic-electric scalpel and the monopolar electrotome are connected to the output device via the signal port so as to obtain the ultrasonic drive signal and the high-frequency electric drive signal.

### Beneficial Effects of the Invention

### Beneficial Effects

According to the technical solution of some embodiments of the present disclosure, the ultrasonic energy source and the high-frequency electric energy source are packaged in the same shell of the output device, so that the output device can simultaneously drive both the ultrasonic surgical instrument and the high-frequency electrosurgical instrument, thereby saving the space of the device, avoiding additional cost, facilitating control device, and helping to improve surgical efficiency and achieve better surgical effects.

### Brief Description of the Drawings

### Brief Description of the Drawings

Other features, objects, and advantages of the disclosure will become more apparent from the following detailed description of non-limiting embodiments when taken in conjunction with the accompanying drawings. In the drawings:
FIG. 1 schematically illustrates a block diagram of an output device for outputting drive signals to a surgical instrument, in accordance with an embodiment of the present disclosure;
FIG 2 schematically illustrates a block diagram of an output device for outputting drive signals to a surgical instrument, in accordance with another embodiment of the present disclosure;
FIG. 3a schematically illustrates a block diagram of an ultrasonic energy source in an output device for outputting drive signals to a surgical instrument, in accordance with another embodiment of the present disclosure;
FIG. 3b schematically illustrates a block diagram of a high-frequency electric energy source in an output device for outputting drive signals to a surgical instrument, in accordance with another embodiment of the present disclosure;
FIG. 4a schematically illustrates a block diagram of an ultrasonic signal acquisition circuit in an output device for outputting drive signals to a surgical instrument, in accordance with another embodiment of the present disclosure;
FIG. 4b schematically illustrates a block diagram of a high-frequency electric signal acquisition circuit in an output device for outputting drive signals to a surgical instrument, in accordance with another embodiment of the present disclosure;
FIG 5 schematically illustrates a block diagram of an output device for outputting drive signals to a surgical instrument, in accordance with another embodiment of the present disclosure;
FIG 6 schematically illustrates a block diagram of a surgical system, in accordance with an embodiment of the present disclosure.

### Detailed Description of the Embodiments

Hereinafter, exemplary embodiments of the present disclosure will be described in detail with reference to the accompanying drawings, so that they can be easily implemented by those skilled in the art. In addition, those parts that are not related to the description of exemplary embodiments are omitted in the drawings for clarity.

In the present disclosure, it should be understood that terms such as "include" or "have", etc. , are intended to indicate the existence of the features, numbers, steps, behaviors, components, parts, or combinations thereof disclosed in the present specification, and are not intended to preclude the possibility that one or more other features, numbers, steps, behaviors, components, parts, parts, or combinations thereof exist or are added.

In addition, it should be noted that, in a case of no conflict, the embodiments in the present disclosure and the features in the embodiments may be combined with each other. The present disclosure will be described below in detail with reference to the accompanying drawings and embodiments.

It should be noted that acquisition or presentation of data in the present disclosure is either authorized by the user, confirmed, or voluntarily selected by the user.

An embodiment of the present disclosure provides an output device for outputting drive signals to a surgical instrument. The output device comprises: an ultrasonic energy source for generating an ultrasonic drive signal; a high-frequency electric energy source for generating a high-frequency electric drive signal, wherein the ultrasonic energy source and the high-frequency electric energy source are located in the same casing; a signal port for connecting to a surgical instrument, so as to provide the ultrasonic drive signal and/or the high-frequency electric drive signal to the surgical instrument; an ultrasonic signal acquisition circuit, used for acquiring and processing signals in a connection circuit between the ultrasonic energy source and the surgical instrument, so as to obtain ultrasonic feedback signals, and providing the ultrasonic feedback signal to a control module, wherein the ultrasonic feedback signals comprise an ultrasonic voltage feedback signal and an ultrasonic current feedback signal; a high-frequency electric signal acquisition circuit, used for acquiring and processing signals in a connection circuit between the high-frequency electric energy source and the surgical instrument, so as to obtain high-frequency electric feedback signals, and providing the high-frequency electric feedback signals to the control module, wherein the high-frequency electric feedback signals comprise a high-frequency voltage feedback signal and a high-frequency current feedback signal; a control module configured to obtain adjustment parameters based on the ultrasonic feedback signals and the high-frequency electric feedback signals; and to control the ultrasonic energy source to output an ultrasonic drive signal and the high-frequency electric energy source to output a high-frequency electric drive signal based on the adjustment parameters; wherein the adjustment parameters comprise a power parameter and a frequency parameter, the power parameter is used for adjusting the power of the ultrasonic drive signal output by the ultrasonic energy source and the power of a high-frequency electric drive signal output by the high-frequency electric energy source, the frequency parameter is used for adjusting the frequency of the ultrasonic drive signal output by the ultrasonic energy source and/or the frequency of the high-frequency electric drive signal output by the high-frequency electric energy source, wherein the surgical instrument comprises one or more of an ultrasonic-electric scalpel, an ultrasonic scalpel, a monopolar electrotome, and a bipolar electrotome.

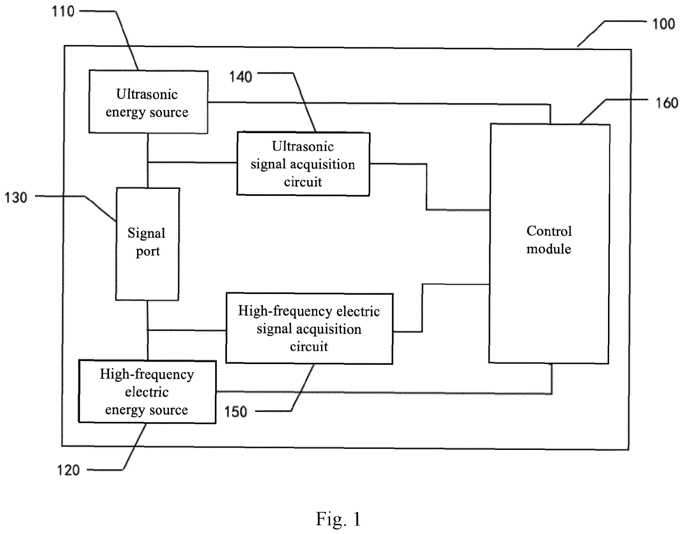

FIG. 1 schematically illustrates a block diagram of an output device for outputting drive signals to a surgical instrument, in accordance with an embodiment of the present disclosure.

As shown in FIG. 1, the output device 100 includes an ultrasonic energy source 110, a high-frequency electric energy source 120, a signal port 130, an ultrasonic signal acquisition circuit 140, a high-frequency electric signal acquisition circuit 150, and a control module 160.

The ultrasonic energy source 110 is used for providing an ultrasonic drive signal for an ultrasonic surgical instrument. The ultrasonic energy source can convert power frequency alternating current into an ultrasonic signal so as to match an ultrasonic transducer used for the ultrasonic surgical instrument, and drive the ultrasonic transducer to convert electric energy into mechanical energy, so as to drive the ultrasonic wave-guide rod of the ultrasonic surgical instrument to vibrate. The frequency used by the ultrasonic surgical instruments for surgical is ranged from 20 to 100 kHz, most commonly 55.5 kHz. The frequency of the signal output by the ultrasonic energy source in embodiments of the present disclosure is also within this range.

A high-frequency electric energy source 120 is used for providing a high-frequency electric drive signal to a high-frequency electric surgical instrument. The frequency used by the high-frequency electric surgical instrument in surgery is ranged from 0.3 to 5 MHz. The term "high frequency" in the present disclosure also refers to a frequency range of 0.3-5 MHz. The frequency of the signal output by the high-frequency electric energy source in the embodiments of the present disclosure also belongs to this range.

According to the embodiments of the present disclosure, the ultrasonic energy source 110 and the high-frequency electric energy source 120 are packaged in a shell of the same output device, that is, are located in the same casing, are connected to a surgical instrument via the signal port 130, and output drive signals to the surgical instrument. There may be several of signal ports 130 for connecting one or more types of surgical instruments. For example, the signal port 130 includes two ports, one port provides drive signals for a ultrasonic-electric scalpel, and this port can output an ultrasonic drive signal and a high-frequency electric drive signal, the other port provides a drive signal for a monopolar electrotome or a bipolar electrotome, and when in use, the control module 160 can automatically control the drive signals output for each signal port 130. In order to ensure stable and normal operation of the currently used surgical instrument, the drive signals output for each signal port 130 may also be manually controlled.. The signal port 130 may also have only one port for connecting a surgical instrument and outputting the drive signal according to requirements, for example, outputting an ultrasonic drive signal when connecting an ultrasonic scalpel, outputting a high-frequency electric drive signal when connecting a monopolar electrotome, and outputting an ultrasonic drive signal and a high-frequency electric drive signal when connecting the ultrasonic-electric scalpel. The output device 100 may also include a signal port for connection with other surgical devices to allow the output device 100 to cooperate with the other surgical devices.

The signal port 130 may also include a port for receiving a manual switch signal of the surgical instrument and providing it to the control module 160. For example, a manual switch is set at the handle of the ultrasonic scalpel, the power level of the ultrasonic scalpel can be selected by means of the manual switch, and the highest output power in the current level is set in each level. In this case, the control module 160 needs to control the output power of the driving signals according to the manual switch signal and the adjustment parameters.

The ultrasonic signal acquisition circuit 140 is used for acquiring and processing signals in a connection circuit between a surgical instrument and the ultrasonic energy source, so as to obtain ultrasonic feedback signals, and providing the ultrasonic feedback signals to the control module 160. The drive signal output by the ultrasonic energy source 110 is a continuous voltage signal. When the surgical instrument touches the biological tissue, a load circuit is formed, and a current signal is generated in the circuit between the surgical instrument and the ultrasonic energy source 110. The ultrasonic signal acquisition circuit 140 synchronously acquires and processes the voltage signal and the current signal in the circuit, and then converts them into discontinuous digital signals, i. e. ultrasonic feedback signals, which are provided for a processor in the control module 160 to perform high-speed digitization processing and computation. The procedure of signal processing in the ultrasonic signal acquisition circuit 140 can reduce the interference components in the signal, and improve the effectiveness and reliability of the operation result.

The high-frequency electric signal acquisition circuit 150 is used for acquiring and processing signals in a connection circuit between the surgical instrument and the high-frequency electric energy source, so as to obtain high-frequency electric feedback signals, and providing the high-frequency electric feedback signals to the control module 160. The high-frequency electric signal acquisition circuit 150 synchronously acquires and processes a voltage signal and a current signal in the circuit, and then converts them into discontinuous digital signals, i. e. high-frequency electric feedback signals, which are provided for a processor in the control module 160 to perform high-speed digitization processing and computation. The procedure of signal processing in the high-frequency electric signal acquisition circuit 150 can reduce the interference components in the signal, and improve the effectiveness and reliability of the operation result.

The control module 160, configured to obtain adjustment parameters based on the ultrasonic feedback signals and the high-frequency electric feedback signals, and based on the adjustment parameters, control the ultrasonic energy source 110 to output an ultrasonic drive signal and control the high-frequency electric energy source 120 to output a high-frequency electric drive signal.

According to an embodiment of the present disclosure, the control module 160 obtains adjustment parameters based on the ultrasonic feedback signals. The feedback signals comprise a voltage feedback signal and a current feedback signal, and accordingly, a phase difference between the voltage signal and the current signal can be obtained. The phase difference can be multiplied by a frequency conversion coefficient of system settings to obtain a frequency offset, and the frequency of the output drive signals can be adjusted according to the frequency offset. On the other hand, an impedance in a load circuit can also be obtained by the feedback signals, and according to the phase difference and the variation of the impedance, the power of the output signals can be precisely adjusted, so that the ultrasonic surgical instrument works with a stable and appropriate power setting.

According to an embodiment of the present disclosure, the adjustment parameters obtained by the control module 160 include a power parameter and a frequency parameter, the power parameter is used for adjusting the power of the ultrasonic drive signal output by the ultrasonic energy source and/or the power of the electric drive signal output by the high-frequency electric energy source, the frequency parameter is used to adjust the frequency of the ultrasonic drive signal output by the ultrasonic energy source and/or the frequency of the electric drive signal output by the high-frequency electric energy source. The frequency of the ultrasonic drive signal needs to be adjusted so as to ensure normal operation of the ultrasonic surgical instrument, the frequency of the electric drive signal generally does not need to be adjusted; and adjusting the power of the ultrasonic drive signal and the power of the electric drive signal is to achieve better surgical effects. In some scenarios, the power of the drive signals do not need to be adjusted, for example, in the case of cutting only or sealing only, or in the case of cutting simple-structured biological tissue, power adjustments may also not be performed during the cutting process.

The control module 160 may control the output of the ultrasonic energy source and the high-frequency electric energy source according to the adjustment parameters, and may also control the output of the energy source in conjunction with other system settings. For example, if a highest power is given by the system settings, the power parameter will give the percentage of the highest power; if a center frequency is given by the system settings, the frequency parameter will provide an offset of the center frequency.

The following describes implementations of determining the power parameter based on the feedback signals.

The impedance in the load circuit can be obtained by the voltage signal V(t) and the current signal I(t), i. e., R(t)=V(t)/I(t). Therefore, using the voltage signal output by the ultrasonic energy source and the current signal in the load circuit can obtain an impedance Rus(t) in the ultrasonic circuit, which is referred to as acoustic impedance for short, and using the voltage signal output by the high-frequency energy source and the current signal can obtain an impedance R_{ES}(t) in the high-frequency electric circuit, which is referred to as electric impedance for short. The cutting process of surgery is tracked by detecting the variations of acoustic impedance and/or electric impedance during the surgical cutting process, and can be used as a basis for adjusting the power of the drive signal, thereby achieving self-adaptive adjustment of the power of the drive signals, and obtaining a better surgical effect. Such impedance may reflect the changed tissue property of the part to be cut currently, for example, protein degeneration, blood coagulation, and the like, so as to match the whole cutting process, and the control module 160 adjusts output power of the drive signals according to the cutting process adaptively. For example, when an ultrasonic-electric scalpel is used to cut liver, the vibration of the ultrasonic scalpel rod can achieve the function of cutting, and the implementation of high-frequency electric energy at the electric jaws can assist coagulation, when increase of the impedance is detected, the output power for driving the ultrasonic-electric scalpel also increases, so as to accelerate the vibration of the ultrasonic scalpel rod. When the impedance is increased, the output power applied to the electric jaws is also increased, so as to improve the efficiency of coagulation or evaporation. In this way, by using the ultrasonic-electric scalpel, the cutting progress can be accelerated, intraoperative bleeding can be reduced, and a better surgical effect can be obtained. For another example, when the small intestine is to be cut, since the tissue has high toughness, the cutting consumes more time, and when the ultrasonic-electric scalpel is used, the output power for driving the ultrasonic-electric scalpe can be increased to accelerate the cutting procedure, and the signal power applied to the electric jaws keeps an appropriate level to evaporate moisture to assist cutting.

According to the embodiment of the present disclosure, the control module 160 is further configured to obtain an acoustic impedance based on the ultrasonic feedback signals and obtain an electric impedance based on the high-frequency power feedback signals; matching the acoustic impedance and/or electric impedance with impedance data to determine a tissue type; and based on the tissue type, matches the acoustic impedance and/or electric impedance with the impedance data to obtain an power parameter. Impedance data, as referred to herein, refers to the impedance characteristics presented by the tissue of a certain type during surgical cutting procedure, the impedance data comprises at least an impedance magnitude and a suggested power value for the surgical instrument used for this tissue. As described above, tracking the cutting process by detecting the variations of acoustic impedance and/or electric impedance in the surgical cutting process can serve as a basis for adjusting the power of the driving signals, achieve self-adaptive adjustment of the power of the driving signals, and obtain a better surgical effect. For example, the characteristic of impedance magnitude presented by cutting the liver and cutting the small intestine is obviously different, and the current tissue type to be cut can be identified by matching the impedance magnitude, so as to find out the power parameter for this tissue.

In addition, the current cutting stage may also be determined by detecting the variations in the rising and falling of the impedance, so as to select an adaptive power in different cutting stages, thereby obtaining a better surgical effect. For example, an impedance characteristic in the initial cutting stage is that the impedance rapidly rises and then falls, a relatively stable impedance is maintained in the cutting-in stage, and in the final cutting stage, the impedance presents a characteristic of rapidly rising. A fixed power setting may be used in the initial cutting stage and the final cutting stage, and the foregoing manner of adaptive adjustment of power is used in the cutting-in stage.

According to an embodiment of the present disclosure, the control module 160 is further configured to: obtain a first phase difference based on the ultrasonic voltage feedback signal and the ultrasonic current feedback signal; obtain a second phase difference based on the high frequency voltage feedback signal and the high frequency current feedback signal; based on the tissue type, match the acoustic impedance and/or the electric impedance with the impedance data, and obtaining the power parameter according to the first phase difference and the second phase difference. According to the phase difference between the voltage signal and the current signal in the load circuit, the power of the signal applied to the load circuit can be adjusted accurately. The actual signal power applied on the surgical instrument is P = UI cos θ. In case of the phase difference θ between the voltage signal and the current signal is not zero, the actual power of the signal will decrease. According to an embodiment of the present disclosure, when get the matched tissue type according to the impedance and obtain the suggested power value, and the phase difference in the current circuit is not zero, the signal power output by the energy source needs to be increased, so as to ensure that the signal power actually applied on the surgical instrument can satisfy the requirements of the suggested power value. For example, if the currently matched tissue type is type 1, correspondingly, the suggested power value of the ultrasonic signal is P1, the suggested power value of the high-frequency electric signal is P2, and the calculated first phase differenceθ₁ at this time, that is, the phase difference of the ultrasonic feedback signal is not zero, while the second phase differenceθ₂ is zero, the control module 160 controls the signal power output by the ultrasonic energy source to be P_{US} = P₁ /cos θ₁, and the signal power output by the high-frequency electric energy source to be P_{ES} = P₂.

The control module 160 may also control the duration of output signals of the ultrasonic energy source and the high-frequency electric energy source. For example, when a ultrasonic-electric scalpel is applied to perform surgical cutting, within a certain period of time, the ultrasonic energy source is controlled to output a drive signal, while the high-frequency electric energy source does not output, and in this case, the ultrasonic-electric scalpel only executes the cutting function of the "ultrasonic scalpel", and within another time period, the high-frequency electric energy source is controlled to output a drive signal, while the ultrasonic energy source does not output, and in this case, the ultrasonic-electric scalpe only executes the coagulation function of the "electrotome", this manner also contributes to a better surgical effect.

According to an embodiment of the present disclosure, the output device 100 may output the drive signal for the surgical instrument including one or more of an ultrasonic-electric scalpel, an ultrasonic scalpel, a monopolar electrotome, and a bipolar electrotome. For example, when an ultrasonic-electric scalpel is used to perform surgery, the output device 100 needs to provide an ultrasonic drive signal and a high-frequency electric drive signal for the ultrasonic-electric scalpe, and when an ultrasonic scalpel and a monopolar electrotome are used to perform surgery, the output device 100 needs to provide an ultrasonic drive signal for the ultrasonic scalpel and provide a high-frequency electric drive signal for the monopolar electrotome.

According to the technical solutions of an embodiment of the present disclosure, the ultrasonic energy source and the high-frequency electric energy source are packaged in a shell of the output device, and the output device can simultaneously drive the ultrasonic surgical instrument and the high-frequency electrosurgical instrument, thereby saving device space, facilitating device control, helping to improve the surgery efficiency and a better surgical effect.

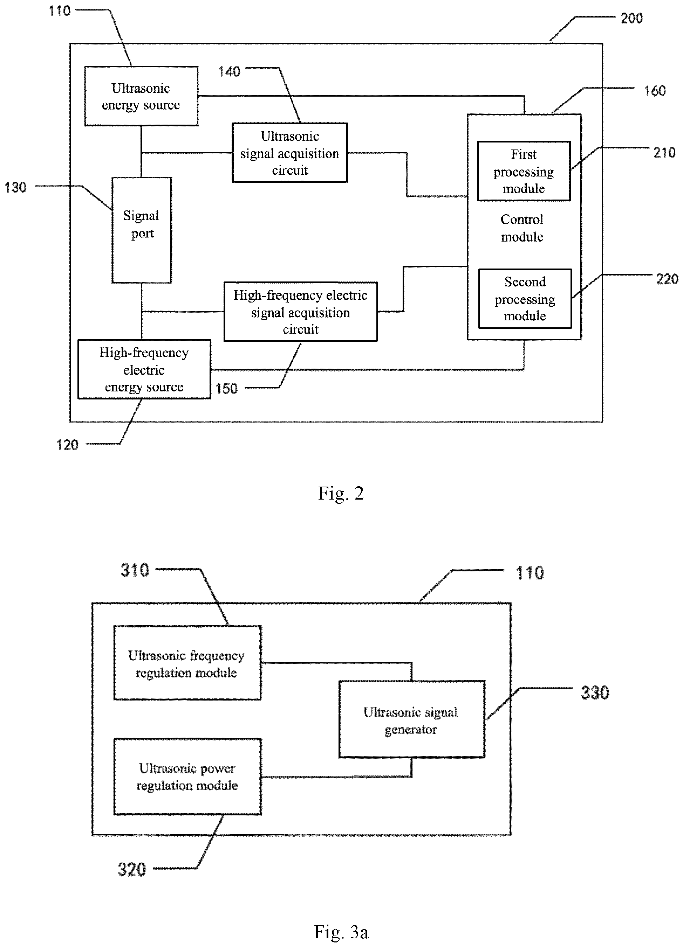

FIG 2 schematically illustrates a block diagram of an output device for outputting drive signals to a surgical instrument, in accordance with another embodiment of the present disclosure..

As shown in FIG. 2, the output device 200 comprises an ultrasonic energy source 110, a high-frequency electric energy source 120, a signal port 130, an ultrasonic signal acquisition circuit 140, a high-frequency electric signal acquisition circuit 150 and a control module 160, wherein the control module 160 comprises a first processing module 210 and a second processing module 220.

The ultrasonic energy source 110, the high-frequency electric energy source 120, the signal port 130, the ultrasonic signal acquisition circuit 140, the high-frequency electric signal acquisition circuit 150, and the control module 160 have functions similar to those of shown if FIG. 1, i.e. the ultrasonic energy source 110, the high-frequency electric energy source 120, the signal port 130, the ultrasonic signal collection circuit 140, the high-frequency electric signal collection circuit 150, and the control module 160 of the output device 100 in the embodiment shown in FIG. 1.

The first processing module 210 is configured to perform digital filtering and digital operation on the ultrasonic feedback signals to obtain an acoustic impedance. By further processing of filtering the ultrasonic feedback signals, the processed feedback signals with lower interference can be obtained; when the processed feedback signals are used to perform phase operation and power operation, the calculation accuracy can be improved, and the accuracy of frequency tracking for the ultrasonic transducer can be improved, thereby ensuring that the output ultrasonic drive signal resonates with the ultrasonic transducer to make the ultrasonic scalpel has the highest work efficiency; meanwhile, as described above, the smaller the error of the phase difference is, the higher the precision of the power adjustment achieves.

The second processing module 220 is configured to perform digital filtering and digital operation on the high-frequency electric feedback signals to obtain an electric impedance. By further processing of filtering the high frequency feedback signals, the processed feedback signals with lower interference can be obtained. When the processed feedback signals are used to perform phase operation and power operation, accuracy of a calculation result can be improved, and more accurate adjustment parameters can be obtained.

According to the embodiment of the present disclosure, the control module 160 may further include a module configured to perform combination analysis processing on the ultrasonic feedback signals and the high-frequency electric feedback signals, so as to perform interference cancellation on the ultrasonic feedback signals, thereby obtaining a more accurate frequency offset.

FIG. 3a schematically illustrates a block diagram of an ultrasonic energy source in an output device for outputting drive signals to a surgical instrument, according to another embodiment of the present disclosure.

As shown in FIG. 3a, the ultrasonic energy source 110 includes an ultrasonic frequency regulation module 310, an ultrasonic power regulation module 320, and an ultrasonic signal generator 330.

The ultrasonic frequency regulation module 310 is used for adjusting the frequency of a signal output by the ultrasonic signal generator;

The ultrasonic power regulation module 320 is used for adjusting the power of a signal output by the ultrasonic signal generator;

The ultrasonic signal generator 330 is used for generating an ultrasonic drive signal corresponding to the frequency and power according to the adjustment of the ultrasonic frequency regulation module 310 and the ultrasonic power regulation module 320.

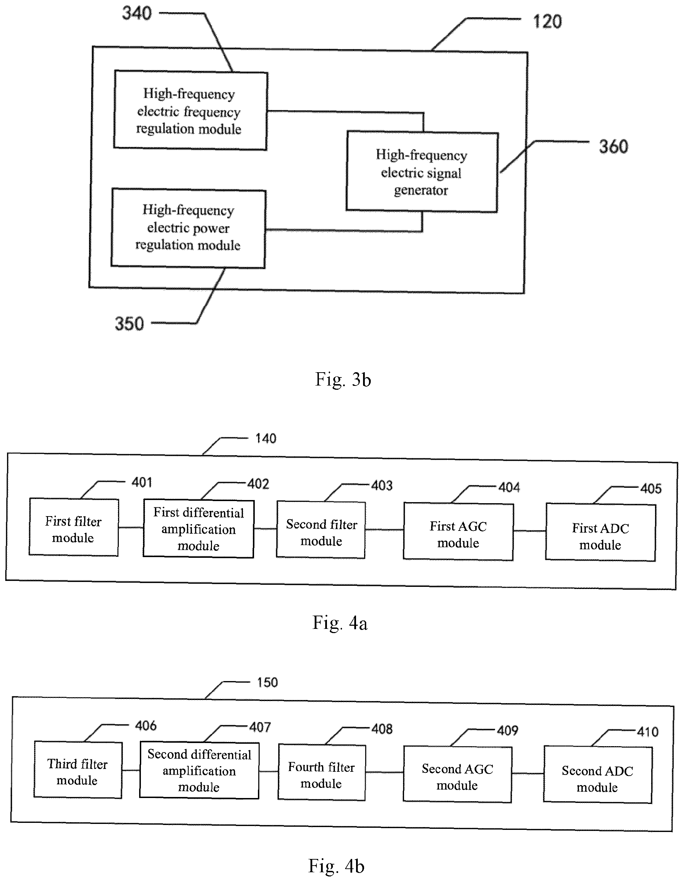

FIG. 3b schematically illustrates a block diagram of a high-frequency electric energy source in an output device for outputting drive signals to a surgical instrument, according to another embodiment of the present disclosure.

As shown in FIG. 3b, the high-frequency electric energy source 120 includes a high-frequency electric frequency regulation module 340, a high-frequency electric power regulation module 350, and a high-frequency electric signal generator 360.

The high-frequency electric frequency regulation module 340 for regulating the frequency of the signal output by the high-frequency electric signal generator;

The high-frequency electric power regulation module 350 for regulating the power of the signal output by the high-frequency electric signal generator;

The high-frequency electric signal generator 360 is used for generating a high-frequency electric drive signal corresponding to the frequency and power according to the adjustment of the high-frequency electric frequency regulation module 340 and the high-frequency power regulation module 350.

The frequency and power of the output signals generated by the ultrasonic energy source and the high-frequency electric energy source are both adjustable in magnitude and switch status according to the embodiment disclosed above with respect to FIG. 3a and FIG. 3b. This can ensure that the surgical instrument can work stably during surgery and can fully exert the advantages of the ultrasonic surgical instrument and the high-frequency electrosurgical instrument.

FIG. 4a schematically illustrates a block diagram of an ultrasonic signal acquisition circuit in an output device for outputting drive signals to a surgical instrument, according to another embodiment of the present disclosure.

As shown in FIG. 4a, the ultrasonic signal acquisition circuit 140 comprises: a first filter module 401, a first differential amplification module 402, a second filter module 403, a first AGC module 404 (AGC being Automatic Generation Control) and a first ADC module 405 (ADC being Analog-to-Digital Converter).

The first filter module 401 is used for performing primary filtering on the acquired ultrasonic signals;

The first differential amplification module 402 is used for performing differential amplification on the primary-filtered signals;

The second filter module 403 is used for performing secondary filtering on the differential-amplified signals;

The first AGC module 404 is used for performing gain control on the secondary-filtered signals to facilitate digital sampling;

The first ADC module 405 is used for performing digital conversion on the processed signals.

FIG. 4b schematically illustrates a block diagram of a high-frequency electric signal acquisition circuit in an output device for outputting drive signals to a surgical instrument, in accordance with another embodiment of the present disclosure.

As shown in FIG. 4b, the high-frequency electric signal acquisition circuit 150 comprises: a third filter module 406, a second differential amplification module 407, a fourth filter module 408, a second AGC module 409 and a second ADC module 410.

The third filter module 406 is used for performing primary filtering on the acquired high-frequency electric signals;
the second differential amplification module 407 is used for performing differential amplification on the primary-filtered signals;
the fourth filter module 408 is used for performing secondary filtering on the differential-amplified signals;
the second AGC module 409 is used for performing gain control on the secondary-filtered signals to facilitate digital sampling;
the second ADC module 410 is used for performing digital conversion on the processed signals.

According to the embodiments disclosed in FIG. 4a and FIG. 4b, the ultrasonic feedback signals and the high-frequency electric feedback signals are obtained by using separate circuits for acquisition and processing, and such framework can reduce interference between the ultrasonic feedback signals and the high-frequency electric feedback signals.

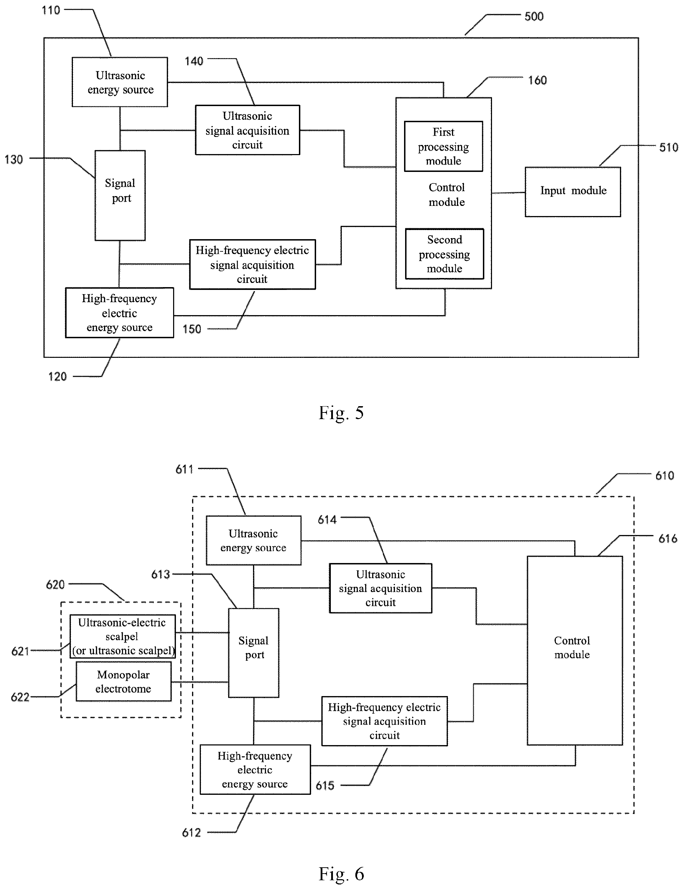

FIG. 5 schematically illustrates a block diagram of an output device for outputting drive signals to a surgical instrument, according to another embodiment of the present disclosure.

As shown in FIG. 5, the output device 500 includes an ultrasonic energy source 110, a high-frequency electric energy source 120, a signal port 130, an ultrasonic signal acquisition circuit 140, a high-frequency electric signal acquisition circuit 150, a control module 160, and an input module 510.

The ultrasonic energy source 110, the high-frequency electric energy source 120, the signal port 130, the ultrasonic signal acquisition circuit 140, the high-frequency electric signal acquisition circuit 150, and the control module 160 have functions similar to those shown in FIG. 1, i.e. the ultrasonic energy source 110, the high-frequency electric energy source 120, the signal port 130, the ultrasonic signal acquisition circuit 140, the high-frequency electric signal acquisition circuit 150, and the control module 160 of the output device 100 in the embodiment shown in FIG. 1.

The input module 510 in the output device 500 is used for receiving parameter settings, and the control module 160 is further configured to control the power of the output signal of the ultrasonic energy source and/or the power of the output signal of the high-frequency electric energy source, according to the parameter settings received by the input module 510 and the adjustment parameters.

According to an embodiment of the present disclosure, the input module 510 may be a digital panel with display and input functions, and is disposed on the outer surface of the case of the output device 500. The input module 510 may also be an external terminal device and is connected to the output device 500 via the signal port 130. For example, when the output device 500 is used, a doctor, using his preliminary determination, sets the maximum power of the high-frequency electrotome or sets a power level combination of the ultrasonic scalpel by means of the input module 510; when the surgery is performed, the control module 160 takes this parameter setting as an upper power limit; and when adaptive power adjustment is performed, as the method described in the foregoing embodiments, the output power also meets the requirements of this parameter setting.

The present disclosure also discloses a surgical system. FIG. 6 schematically shows a block diagram of a surgical system according to an embodiment of the present disclosure.

As shown in FIG. 6, the surgical system comprises an output device 610 and a surgical instrument 620, wherein the output device 610 comprises an ultrasonic energy source 611, a high-frequency electric energy source 612, a signal port 613, an ultrasonic signal acquisition circuit 614, a high-frequency electric signal acquisition circuit 615 and a control module 616.

The ultrasonic energy source 611 is used for outputting an ultrasonic driving signal.

The high-frequency electric energy source 612 is used for outputting a high-frequency electric drive signal; and the ultrasonic energy source 611 and the high-frequency electric energy source 612 are located in the same casing.

The signal port 613 is used for connecting to the surgical instrument 620, so that the surgical instrument 620 obtains an ultrasonic drive signal output by the ultrasonic energy source 611 and a high-frequency electric drive signal output by the high-frequency electric energy source 612.

The ultrasonic signal acquisition circuit 614 is used for acquiring and processing signals in a connection circuit between the surgical instrument and the ultrasonic energy source 611, so as to obtain ultrasonic feedback signals, and providing the ultrasonic feedback signals to the control module 616, wherein the ultrasonic feedback signals comprise an ultrasonic voltage feedback signal and an ultrasonic current feedback signal.

The high-frequency electric signal acquisition circuit 615, is used for acquiring and processing signals in an connection circuit between the surgical instrument and the high-frequency electric energy source 612, so as to obtain high-frequency electric feedback signals, and providing the high-frequency electric feedback signals to the control module 616, wherein the high-frequency electric feedback signals comprise a high-frequency voltage feedback signal and a high-frequency current feedback signal.

The control module 616 is configured to obtain adjustment parameters based on the ultrasonic feedback signals and the high frequency feedback signals; and control the ultrasonic energy source 611 to output an ultrasonic drive signal and control the high-frequency electric energy source 612 to output a high-frequency electric drive signal, based on the adjustment parameters; the adjustment parameters comprise a power parameter and a frequency parameter, wherein the power parameter is used for adjusting the power of the ultrasonic drive signal output by the ultrasonic energy source 611 and the power of the high-frequency electric drive signal output by the high-frequency electric energy source 612, and the frequency parameter is used for adjusting the frequency of the ultrasonic drive signal output by the ultrasonic energy source 611 and/or the frequency of the high-frequency electric drive signal output by the high-frequency electric energy source 612.

The surgical instrument 620 includes an ultrasonic-electric scalpel (or ultrasonic scalpel) 621 and a monopolar electrotome 622, and the ultrasonic-electric scalpel 621 and monopolar electrotome 622 are connected to the output device 610 via the signal port 613, so as to obtain the ultrasonic drive signal and the high-frequency electric drive signal.

The output device 610 in the surgical system according to the embodiment of the present disclosure has all functions provided by the output device 100 in the foregoing embodiments, and a repeated description thereof will be omitted here.

The foregoing description is merely illustrative of the preferred embodiments of the present disclosure and of the technical principles applied thereto. It should be understood by those skilled in the art, the scope of the present disclosure is not limited to the technical solution formed by the specific combination of the described technical features, the present disclosure should also cover other technical solutions formed by any combination of the above technical features or their equivalent features without departing from the inventive concept. For example, technical solutions formed by replacing the above features with (but not limited to) technical features having similar functions disclosed in the present disclosure.

## Claims

1. An output device for outputting drive signals to a surgical instrument, comprising:
an ultrasonic energy source for generating an ultrasonic drive signal;
a high-frequency electric energy source for generating a high-frequency electric drive signal, wherein the ultrasonic energy source and the high-frequency electric energy source are located in the same casing;
a signal port for connecting to a surgical instrument, so as to provide the ultrasonic drive signal and/or the high-frequency electric drive signal to the surgical instrument;
an ultrasonic signal acquisition circuit, used for acquiring and processing signals in a connection circuit between the ultrasonic energy source and the surgical instrument, so as to obtain ultrasonic feedback signals, and providing the ultrasonic feedback signals to a control module, wherein the ultrasonic feedback signals comprise an ultrasonic voltage feedback signal and an ultrasonic current feedback signal;
a high-frequency electric signal acquisition circuit, used for acquiring and processing signals in a connection circuit between the high-frequency electric energy source and the surgical instrument, so as to obtain high-frequency electric feedback signals, and providing the high-frequency electric feedback signals to the control module, wherein the high-frequency electric feedback signals comprise a high-frequency voltage feedback signal and a high-frequency current feedback signal;
a control module configured to obtain adjustment parameters based on the ultrasonic feedback signals and the high-frequency electric feedback signals; and to control the ultrasonic energy source to output an ultrasonic drive signal and the high-frequency electric energy source to output a high-frequency electric drive signal based on the adjustment parameters; wherein the adjustment parameters comprise a power parameter and a frequency parameter, the power parameter is used for adjusting the power of the ultrasonic drive signal output by the ultrasonic energy source and the power of a high-frequency electric drive signal output by the high-frequency electric energy source, the frequency parameter is used for adjusting the frequency of the ultrasonic drive signal output by the ultrasonic energy source and/or the frequency of the high-frequency electric drive signal output by the high-frequency electric energy source,
wherein the surgical instrument comprises one or more of an ultrasonic-electric scalpel, an ultrasonic scalpel, a monopolar electrotome, and a bipolar electrotome.

2. The output device for outputting drive signals to a surgical instrument according to claim 1, wherein the control module obtains adjustment parameters based on the ultrasonic feedback signals and the high-frequency electric feedback signals, including:
obtains an acoustic impedance based on the ultrasonic feedback signals, and obtains an electric impedance based on the high-frequency electric feedback signal;
matches the acoustic impedance and/or the electric impedance with impedance data to determine a tissue type;
based on the tissue type, matches the acoustic impedance and/or the electric impedance with the impedance data to obtain a power parameter.

3. The output device for outputting drive signals to a surgical instrument according to claim 2, the control module is further configured to:
obtain a first phase difference based on the ultrasonic voltage feedback signal and the ultrasonic current feedback signal;
obtain a second phase difference based on the high-frequency voltage feedback signal and the high-frequency current feedback signal;
based on the tissue type, match the acoustic impedance and/or the electric impedance with the impedance data, and obtain the power parameter according to the first phase difference and the second phase difference.

4. The output device for outputting drive signals to a surgical instrument according to claim 1, wherein the control module is further configured to control the duration of output signals of the ultrasonic energy source and the high-frequency electric energy source.

5. The output device for outputting drive signals to a surgical instrument according to claim 1, wherein the control module further comprises:
a first processing module, configured to perform digital filtering and digital operation on the ultrasonic feedback signals to obtain an acoustic impedance;
a second processing module, configured to perform digital filtering and digital operation on the high-frequency electric feedback signals to obtain an electric impedance.

6. The output device for outputting drive signals to a surgical instrument according to claim 1, wherein,
the ultrasonic energy source comprises: an ultrasonic frequency regulation module, an ultrasonic power regulation module and an ultrasonic signal generator;
the high-frequency electric energy source comprises: a high-frequency electric frequency regulation module, a high-frequency electric power regulation module and a high-frequency electric signal generator.

7. The output device for outputting drive signals to a surgical instrument according to claim 1, wherein the ultrasonic signal acquisition circuit comprises: a first filter module, a first differential amplification module, a second filter module, a first automatic-gain-control module, and a first analog-to-digital conversion module.

8. The output device for outputting drive signals to a surgical instrument according to claim 1, wherein the high-frequency electric signal acquisition circuit comprises: a third filter module, a second differential amplification module, a fourth filter module, a second automatic-gain-control module, and a second analog-to-digital conversion module.

9. The output device for outputting drive signals to a surgical instrument according to claim 1, wherein the signal port is further used for receiving a manual switch signal of the surgical instrument and providing the manual switch signal to the control module;
and the control module is further configured to control the power of an output signal of the ultrasonic energy source and/or the power of an output signal of the high-frequency electric energy source according to the manual switch signal and the adjustment parameters.

10. The output device for outputting drive signals to a surgical instrument according to claim 1, further comprising an input module for receiving parameter settings;
and the control module is further configured to control the power of an signal output by the ultrasonic energy source and/or the power of an signal output by the high-frequency electric energy source according to the parameter settings and the adjustment parameters.

11. A surgical system, comprising an output device and a surgical instrument,
the output device comprises:
an ultrasonic energy source for outputting an ultrasonic drive signal;
a high-frequency electric energy source for outputting a high-frequency electric drive signal, wherein the ultrasonic energy source and the high-frequency electric energy source are located in the same casing;
a signal port for connecting to a surgical instrument, so that the surgical instrument obtains the ultrasonic drive signal and/or the high-frequency electric drive signal;
an ultrasonic signal acquisition circuit, used for acquiring and processing signals in a connection circuit between the ultrasonic energy source and the surgical instrument, so as to obtain an ultrasonic feedback signal, and provide the ultrasonic feedback signals to a control module, wherein the ultrasonic feedback signals comprise an ultrasonic voltage feedback signal and an ultrasonic current feedback signal;
a high-frequency electric signal acquisition circuit, used for acquiring and processing signals in a connection circuit between the high-frequency electric energy source and the surgical instrument, so as to obtain high-frequency electric feedback signals, and providing the high-frequency electric feedback signals to a control module, wherein the high-frequency electric feedback signals comprise a high-frequency voltage feedback signal and a high-frequency current feedback signal;
a control module configured to obtain adjustment parameters based on the ultrasonic feedback signals and the high-frequency electric feedback signals; and to control the ultrasonic energy source to output an ultrasonic drive signal and the high-frequency electric energy source to output a high-frequency electric drive signal based on the adjustment parameters; wherein the adjustment parameters comprise a power parameter and a frequency parameter, the power parameter being used for adjusting the power of the ultrasonic drive signal output by the ultrasonic energy source and the power of the high-frequency electric drive signal output by the high-frequency electric energy source, the frequency parameter being used to adjust the frequency of the ultrasonic drive signal output by the ultrasonic energy source and/or the frequency of the high-frequency electric drive signal output by the high-frequency electric energy source;
the surgical instrument comprises an ultrasonic-electric scalpel and a high-frequency electrotome, wherein the ultrasonic-electric scalpel and the high-frequency electrotome are connected to the output device via the signal port, so as to obtain an ultrasonic drive signal and a high-frequency electric drive signal; the high-frequency electrotome is one or both of monopolar electrotome and bipolar electrotome.
